# EUROPEAN PATENT APPLICATION

(11) **EP 0 592 734 A1**
(43) Date of publication of application: **20.04.1994**
(21) Application number: 92309319.9
(22) Date of filing: 13.10.1992
(51) Int. Cl.: A43B 13/40, A43B 7/28

(54) **Adjustable orthotic**

(71) Applicant: Kellerman, David, Santa Barbara, California 93109 (US)
(72) Inventor: Kellerman, David, Santa Barbara, California 93109 (US)
(74) Representative: Thomson, Paul Anthony

(57) **Abstract**

A shoe insert that can be customized by the user to control pronation of the foot and to relieve or reduce stress at painful areas of the foot comprising a film of plastic (126) capable of being deformed to the shape of the foot by the weight of the user. The film includes fastening hook or loop material on at least one surface (122,124) whereby detachable cushioning elements (80,100) can be attached to the film. The cushioning element (80,100) is preferably a sheet of loop cloth that continuously covers one or both surfaces of the film. The cushioning pads (80,100) also contain a sheet of loop or hook material on one or both surfaces. The pads can be inclined to affect pronation or can contain apertures to relieve stress. The outer edges of the pads and the upper edge of the aperture are preferably chamfered or rounded to reduce discomfort.

## Description

### Technical Field

The present invention relates to insoles for shoes and, more particularly, this invention relates to an adjustable orthotic by which the eversion and abduction of the tarsal and metatarsal joints can be controlled by the user.

### Background of the Invention

It has been recognized that many of the minor and major traumatic conditions of the foot, leg and knee can be caused by the misalignment of the joints in the foot. These conditions can be hereditary such as the presence of a longer or shorter leg, can be acquired through traumatic injury or can be caused by repetitive stress or strain on the joints experienced during industrial or athletic activity.

Misalignment of the foot can cause minor trauma such as inflammation, blisters, rashes, calluses, corns, ingrown toe nails or more aggravated conditions such as bunions or bone spurs, typically in the heel area. Over-the-counter insoles are available to relieve minor discomfort. However, they are available in fixed sizes and thicknesses. It is possible to reduce discomfort and promote healing of inflamed areas by custom-cutting thick cushioning products such as Moleskin® into pads which can be locally adhered to the traumatic area or adjacent to the traumatic area. Pads and insoles only treat the symptoms of the misalignment and simply reduce irritation and attempt to prevent further trauma by reducing pressure, rubbing, or abrasion on the sensitive area of the foot. Sometimes surgery is utilized to remove the calluses, corns or bunions.

A more scientific approach has been to develop customized biomechanical devices that are intended to correct the misalignment of the joints. These devices, known as orthotics, are prescribed by medical specialists such as orthopedic doctors, podiatrists or doctors specializing in sports medicine. After extensive physical measurements of the patient, a plaster impression of the foot is taken. The orthotic is manufactured from the impression to specifications provided by the doctor as to the degree of pronation correction required. A soft layer of foam can be applied to the top or bottom surface of the orthotic to provide comfort and to prevent the orthotic from sliding when in a shoe.

Orthotics are very expensive, on the order of several hundred dollars a pair. Since they are so expensive, they are usually designed to provide the final correction. The degree of correction can be so severe that the orthotic causes such pain and discomfort to the patient, that it discourages usage of the orthotic and eventually it is no longer used. It is not possible to adjust the fit of the hard plastic orthotic.

### Statement of the Prior Art

Borsiert, et al. (U.S. Patent No. 4,813,157) discloses a shoe insert in which the thickness of the arch region of a shoe insert can be changed by peeling off superimposed films of padding material. This product is not found on the market. This is probably because the films are adhesively secured and residual adhesive can transfer to socks and /or collect on the surface of the permanent insole. The thickness adjustment is limited to one area of the insole.

Greenwalt (U.S. Patent No. 4,694,590) discloses an arch support unit containing preformed resilient elements located in the arch area. The support is fastened to the shoe by hook and loop fastener elements located in the heel portion of the support and adhered to the support and to the insole of the shoe. The heel cushion disclosed by Scheuerman (U.S. Patent No. 4,928,404) contains a soft insert of silicone rubber opposite the location of a heel spur in the patients heel.

Andrews (U.S. Patent No. 4,793,078) discloses a molded, resilient foam shoe insert having depressions 18 in the heel and in the arch support region. The depressions can be filled with adhesively secured, resilient plastic inserts 19 or may be left empty as dictated by the comfort of the wearer.

Engle (U.S. Patent No. 4,930,232) discloses a multilayer laminated permanently adhered shoe insole formed of materials of different shore hardness.

Kristan (U.S. Patent No. 2,928,193) discloses a composite leather-cork shoe lining filled with resilient pads of sponge or with foam rubber glued to the lining. Bittner (U.S. Patent No. 3,143,812) discloses a thermoplastic insole that can be sized to shape by tearing off portions of the sole along heat sealed seams.

The prior insoles were mainly designed to alleviate pain and discomfort. Most of these products were formed of soft resilient materials having no memory or very hard, rigid materials that must be preformed to a desired shape. Adjustable thickness is provided only in set locations and usually by removing pads to form cushions opposite painful areas of the foot.

An insole having provision for placement of pads of varying thickness anywhere along the bottom surface of an insole is disclosed and claimed in application Serial No. 07/690,661, filed April 24, 1991, the disclosure of which is expressly incorporated herein by reference. The insole is formed of a deformable plastic with memory. The bottom surface contains longitudinal strips of loop or hook material along the bottom surface of the insole. The surface may also contain an index scale for locating the correct place to position a pad.

### Statement of the Invention

The present invention provides a further improvement in the design of an insole that allows it to perform as an orthotic. The insole can be modified by the user to contain elevated and/or relieved areas anywhere on the insole to form an orthotic-like device. By trial and error placement of pads of varying thickness on the bottom surface, letting comfort or discomfort be the guide, the user can create a customized therapeutic device capable of relieving pain and stress and capable of biomechanically correcting or alleviating misaligned conditions in the patient's foot. More expert alignment can be provided by a doctor of medicine trained in correcting misalignment of the feet.

The orthotic of the invention provides infinite adjustability in the location and thickness of the orthotic at any location. The orthotic can be adjusted and shaped at a doctor's clinic. The custom-shaped orthotic can be the permanently prescribed orthotic or can be a temporary device until a permanent orthotic is fabricated. The custom-configured orthotic can be shaped to provide only a fraction of the correction initially and the pads can be increased or decreased in thickness and/or location to provide gradual and more comfortable correction. The patient can be provided with a supply of pads of varying thickness and be allowed to change the pads under supervision of the clinic without the expense and inconvenience of numerous visits to the physician.

The insole of the invention is designed to permit the pads to be secured to the top or bottom surface. Since feet are symmetrical this allows the same insole to be used on the right or left foot permitting sale of single insoles. The tooling and manufacturing costs are significantly reduced.

The invention also includes specially shaped inclined ramps which when appropriately located provide pronation correction. The invention also provides pads with all edges smoothed to provide comfort to the user. The invention also relates to use of improved materials and manufacturing processes to form the insole.

These and other features and many attendant advantages of the invention will become apparent as the invention becomes better understood by reference to the following detailed description when considered in conjunction with the accompanying drawings.

### Brief Description of the Drawings

Figure 1 is a view in section of an adjustable shoe insert according to the invention;
Figure 2 is a view in section taken along line 2-2 of Figure 1;
Figure 3 is a view in elevation taken in the direction of lines 3-3 of Figure 1;
Figure 4 is a view in section of another embodiment of an adjustable shoe insert;
Figure 5 is a view in elevation of the shoe insert of Figure 4 shown with the lowermost layer being peeled away;
Figure 6 is a view in section of the shoe insert of Figure 4 illustrated being inserted into a shoe;
Figure 7 is a view in section of a thick pad for attachment to the shoe insert of the invention;
Figure 8 is a view in section of a thin pad for attachment to the shoe inserts of the invention;
Figure 9 is a view in section of an inclined pad;
Figure 10 is a view in section of a pad having rounded outer edges;
Figure 11 is a view in section of a stacked assembly at a plurality of pads;
Figure 12 is a view in section of a pad having a cavity disposed in the metatarsal region;
Figure 13 is a view in section of a pad having a cavity over the heel region;
Figure 14 is a view in elevation showing the assembly of an inclined pad to the top surface a shoe insert;
Figure 15 is a view in section taken along line 15-15 of Figure 14;
Figure 16 is a view in section showing an inclined pad in dotted lines attached to the opposite side of a shoe insert; Figure 17 is a view in section taken along line 17-17 of
Figure 16;

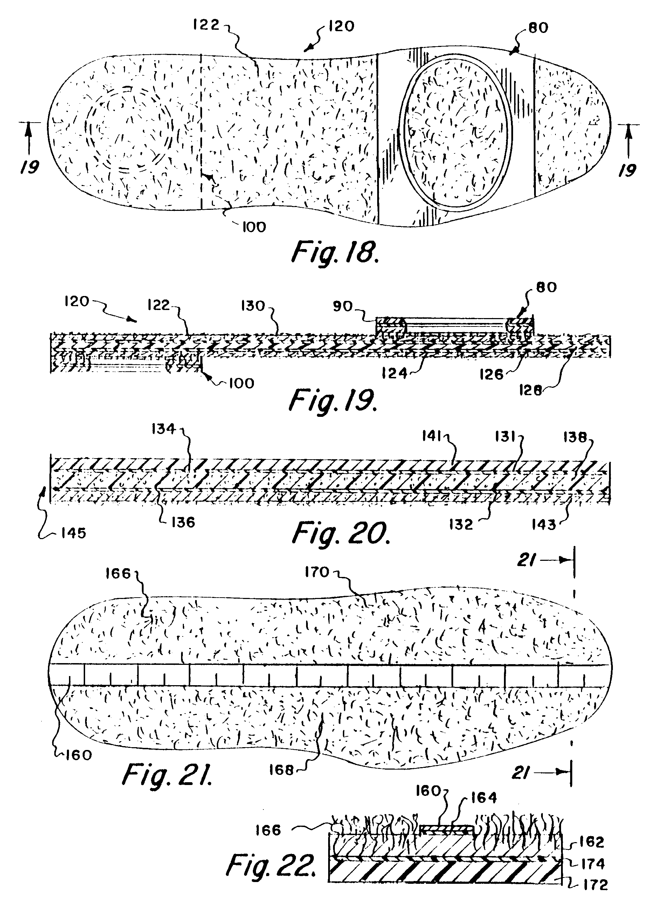

Figure 18 is a view in elevation showing attachment of pads to the metatarsal and heel regions of a double-sided shoe insert;
Figure 19 is view in section taken along line 19-19 of Figure 18;
Figure 20 is a view in section of a further embodiment of a shoe insert including a resilient layer;
Figure 21 is a view in elevation of a still further embodiment of a shoe insert containing a location guide;
Figure 22 is a view in section taken along line 22-22 of Figure 21; and

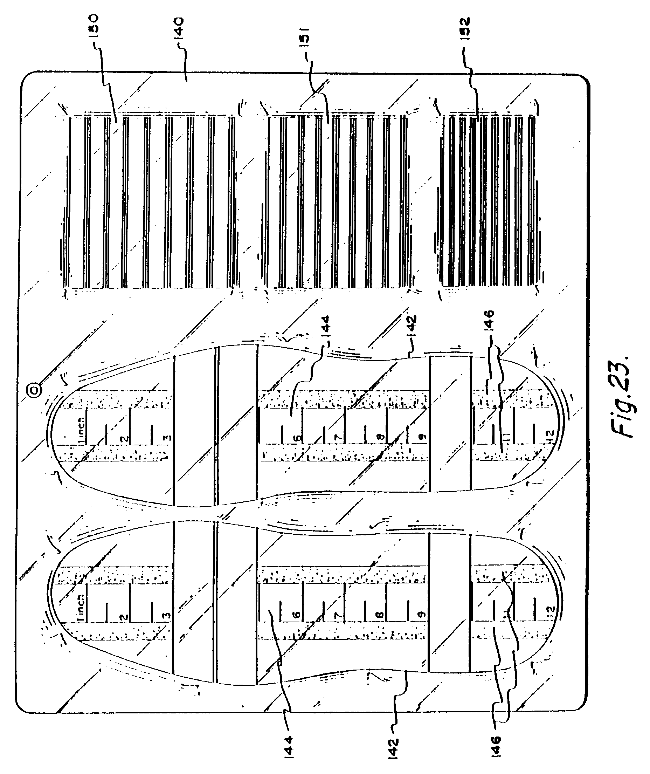

Figure 23 is a plan view showing a kit of a shoe insert and cushion pad elements of varying thicknesses.

### Detailed Description of the Drawings

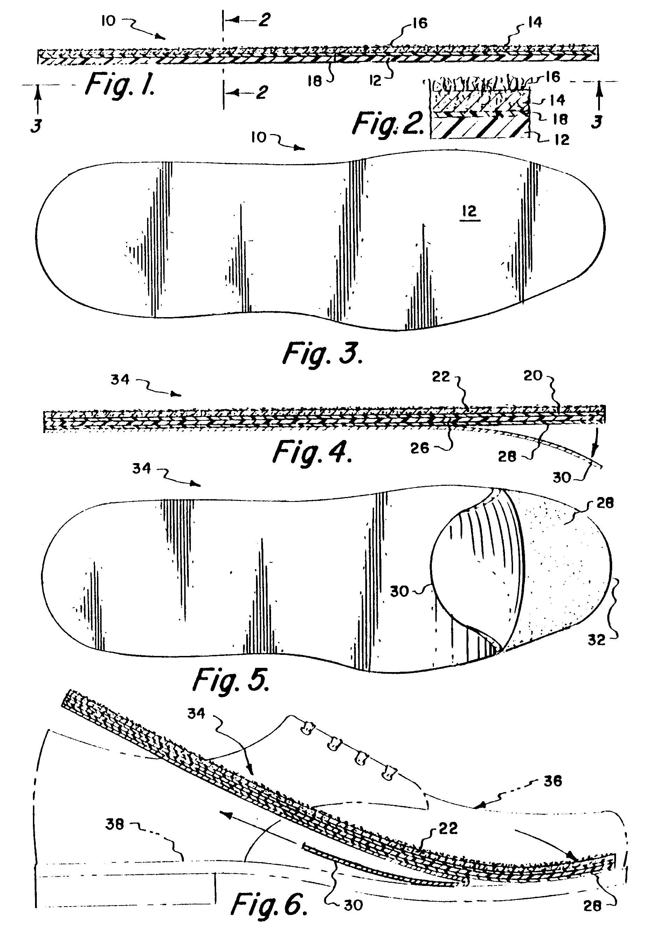

Referring now to Figures 1-3, the shoe insert 10 of the invention is a multilayer laminate in the shape of a shoe insole. The insert 10 comprises a base 12 and an outer pad attaching layer 14. The layer is preferably formed of a cloth having loops 16 to which pads having a layer of hooks can attach. The cloth can form the upper surface of an insole or be disposed toward the bottom surface. The layer 14 is attached to the base 12 by bonding, suitably by means of a layer 18 of adhesive. Optionally a cushioning layer 20 of closed cell foam or other elastomeric material can also be present.

The base is preferably formed of a flexible but deformable plastic with memory such as 10-100 mil high density polyethylene (HDPE), generally from 20-50 mil. Under the force of the weight of the user, the HDPE layer will deform and mold to the shape of the foot. HDPE also presents a low friction surface when used as the upper surface of the insert. The smooth surface reduces irritation and can result in reduction of inflammation and swelling.

The cloth layer is preferably formed of a synthetic resin such as a Nylon polyamide and contains a loop surface adapted for releasable engagement by hooks. The loop cloth is commercially available laminated to foam backing layers such as polyester or polyurethane foam cores in various thicknesses. These materials have been used in industrial applications such as display panels office partitions, bulletin boards, etc. These loop fabrics are washable with water and detergent or can be cleaned with organic solvent to remove common stains. An example of a commercial loop cloth is Veltex Bright materials.

The Veltex nylon cloth materials have a thin, low profile, will not fray and will not pick up lint. They are available in many colors and can be attached by pressure sensitive adhesives, tapes, ultrasonic, or thermal bonding.

Referring now to Figures 4-6, a cloth layer 22 and foam layer 20 are adhered to the deformable base 26. The lower surface of the base contains a layer 28 of adhesive covered by a protective sheet 30. As the sheet 30 is peeled from the top edge 32 of the shoe insert 34, the adhesive layer 28 is exposed. When the insert is placed in a shoe 36, the adhesive layer 28 will adhere to the surface 38 of the insole. The protective sheet 30 is then removed. The cloth layer 22 is exposed for adherence of pads containing cooperatively engaging hook material.

The preferred hook materials used with the pads are also preferably formed of a thin, low profile, non-frayable material. The hooks can form bonds with the loop material of from 20 to 100 pounds per inch, usually around 60-80 pounds per inch. Preferred materials have good elongation properties. Again the preferred hook materials are formed of Nylon. They can be precoated with adhesive. A suitable commercial material is ULTRA-MATE hook materials.

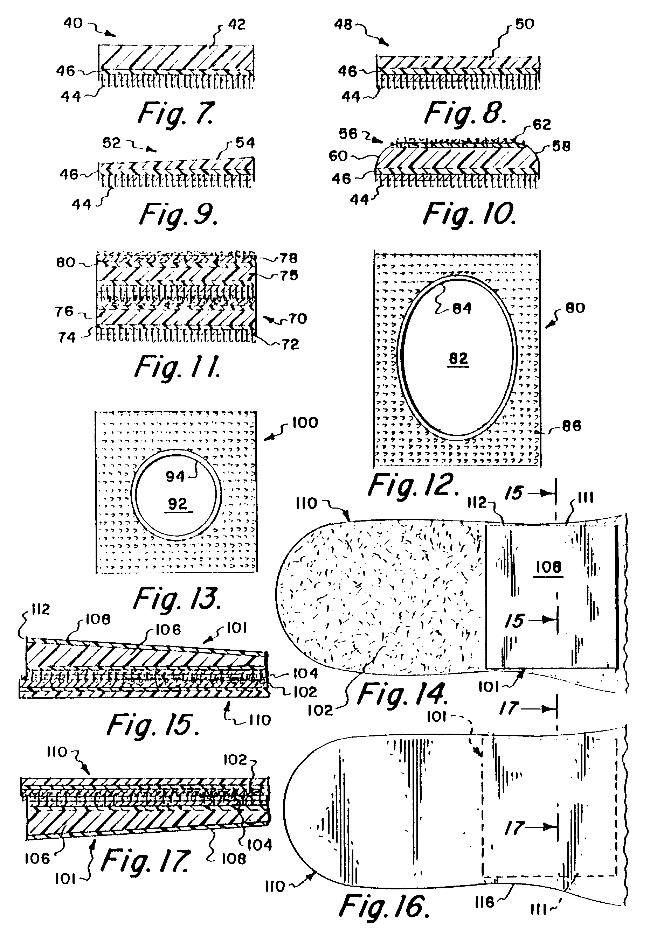

Various pad configurations are illustrated in Figures 7-13. Each pad contains a cushioning layer formed of a resilient material, suitably a closed cell foam such as PORON. The pads can vary in thickness but generally are from about 1/8 inch to about 1/2 inch in thickness. The hook material is bonded to the cushioning layer by adhesive, thermal or ultrasonic bonding or by adhesive tape.

Referring now to Figure 7, a thick pad element 40 has a cushioning layer 42 attached to a layer 44 of hook material by a film 46 of adhesive. The thin pad element 48 shown in Figure 8 contains a thinner layer 50 of cushioning material. The pronation pad 52 illustrated in Figure 9 includes an inclined cushioning layer 54. The pad 56 illustrated in Figure 10 has rounded edges 58, 60 and can include a top layer 62 of hook or loop material so that pads can be stacked as shown in Figure 11.

In Figure 11 the lowermost pad 70 has a bottom hook layer 72 attached by adhesive film 74 to a cushioning layer 76 and an upper loop cloth layer 78 attached to the cushioning layer 75 by an adhesive film 80.

Figures 12 and 13 illustrate pads with apertures which can relieve pressure when placed under sensitive areas of a foot. The larger pad 80 shown in Figure 12 has a large oval aperture 82 cut out of the middle of the pad. The edge 84 of the cut-out is preferably smoothed or chamfered to provide comfort to the user as his foot enters the aperture 82. The attachment surface is formed of detachable material such as hook cloth 86 or loop cloth, not shown. The pad 80 can contain an internal layer 8 of cushioning material, not shown, and an outer layer 90 which can be a smooth cloth such as loop cloth as shown in Figures 17 and 18.

The smaller pad 100 shown in Figure 13 has a circular opening 92, otherwise the construction is identical to that of pad 80. The pad 100 is intended to be placed over inflammations on or in the heel of the user such as a heel spur. Again the edge 94 is chamfered for comfort. The pad can be cut in half and trimmed by the user to form a smaller opening. The two halves are reassembled by placing the openings adjacent each other with the hooks engaging the loops present on the surface of a shoe insert.

The pads can be attached to the top surface or bottom surface of the shoe insert. Referring now to Figures 14-17, inclined or ramp pad 101 is attached to the loop cloth surface 102 of a shoe insert 110. The pad 101 has a bottom hook cloth layer 104, and inclined shaped cushioning layer 106 and an upper smooth layer 108 such as loop cloth. In Figures 14 and 15, the shoe insert 102 is disposed with the loop cloth surface 104 facing upwardly. The pad 101 is attached across the arch and metatarsal area with the thicker edge 112 disposed along the outer edge 111 of the foot. This lift will pronate the foot inwardly to correct bow leg conditions.

The loop surface 102 of the shoe insert 110 is disposed downwardly in Figures 16 and 17. The hook layer 104 of the pad is attached to the loop cloth 102 with the thicker edge 111 disposed among the inner side edge 116 of the shoe insert along the arch-metatarsal region of the foot. This assembly will pronate the foot outwardly to correct a fallen arch or knock-knee condition.

Rather than inverting the shoe insert base, the base 120 as shown in Figures 18 and 19 can have a loop cloth layer 122, 124 on both the top and bottom surfaces. The cloth layers 122, 124 are adhered to a deformable film 126 of HDPE or other resin by adhesive layers 128, 130. Cushioning layers, not shown, may be present beneath the cloth layers.

A metatarsal pad 80 can be attached to the top loop cloth layer 122 and a heel pad 100 can be attached to the bottom loop cloth layer 124, as shown.

Figure 20 illustrates the use of pressure sensitive adhesive transfer tapes to manufacture the pad-receiving base for the shoe insert. Transfer tapes are marketed in the form of a thin strip of pressure-sensitive adhesive preapplied to a release liner and wound into a spiral on a hub. The transfer tapes are preferred over liquid adhesives, hot melt adhesives, staples, waxes or thermal or flame bonding. Heat may distort the cushioning layer or the HDPE base.

As the strip of adhesive is released from the liner it is sticky on both surfaces. It is readily automated to be used in continuous manufacturing operations. It is easy to quickly apply a neat precise strip of clean, dry adhesive strips 131, 132 such as 3MF-927 transfer tape to both sides 134, 136 of cushioning layer 138 such as a 62 mil thick layer of Poron resilient material.

By feeding the adhesive coated cushioning layer 138, a 20 mil thick film of polyethylene 141 and a continuous strip 143 of Veltex Bright loop cloth, a laminate 145 having the cross-section of Figure 20 is formed. The shoe insert bases are then die cut from the laminate.

As an alternative, the loop cloth layer can be flame bonded to the cushioning material as a first operation. The cloth covered cushion layer can be adhesively bonded to the HDPE plastic base by means of adhesive transfer strips. Another alternative is to apply a second layer of loop cloth or loop cloth-cushion laminate to the other surface of the base.

Figure 21-22 illustrates another embodiment of the invention. The pads must be precisely located. If they intrude under an inflamed area, it is very painful. To aid the user in locating the position, a visual index scale 160 can be bonded to the cushioning layer 162 by a film of adhesive 164 or to the cloth layer 166. When the scale 160 is bonded to the cushioning layer the cloth layer can be divided into 2 segments 168, 170 on each side of the scale 160. The scale could also be printed or silk-screened directly onto the cloth layer 166. A film 172 of deformable plastic is bonded by a film 174 of transfer tape or other adhesive means to the other surface of the cushioning layer 162.

The use of strips of hook or loop material instead of a continuous layer is illustrated in Figure 23.

A good way to deliver the insert to the user is shown in Figure 23. A blister package 140 includes a pair of low friction insert 142, each having an index scale 144 thereon, with flanking strips 146 of adhesive or Velcro type fastening material. The user may use the scale to establish and maintain the discrete and movable cushion elements at the best locations that he discover with use. Normally the insert would be supplied fully covered with elements, but only three per insert are shown in Figure 23 to help make the scale visible. Three groups of replacement cushion elements 150, 151, and 152 are also included that have a variety of thicknesses to help custom shape the insert. Even if the users foot shape requirements change with time, the kit of Figure 23 allows continuing modification of the insert to an optimal configuration.

It is to be realized that only preferred embodiments of the invention have been described and that numerous substitutions, modifications and alterations are permissible without departing from the spirit and scope of the invention as defined in the following claims.

## Claims

1. A shoe insert comprising in combination
a thin sheet of plastic shaped to fit over the inside surface of a shoe, said plastic capable of deforming to the shape of the foot of the user,
the first surface of said sheet including lap or hook fabric fastening material whereby cushion pad elements having a hook or loop surface can be attached to said material to selectively modify the thickness or pitch of said insert from the toe and to the heel end of the insert.

2. An insert according to claim 1 in which the fastening material is provided as at least one strip extending substantially from the toe end to the heel end of said surface.

3. An insert according to Claim 1 in which said fastening material is provided on both surfaces of said sheet.

4. An insert according to Claim 3 in which the fastening material is a sheet of loop cloth continuously covering both said surfaces.

5. An insert according to Claim 1 in which the fastening material is a sheet of loop cloth continuously covering said shaped sheet.

6. An insert according to Claim 5 further including a layer of cushioning material disposed between said sheet and said fastening material.

7. An insert according to Claim 5 in which the other surface of said shaped sheet includes a layer of adhesive and a peelable protective film applied to the layer of adhesive.

8. An insert according to Claim 5 in which said fastening material is thermally bonded to said layer of cushioning material.

9. An insert according to Claim 5 in which the fastening material is adhesively bonded to said cushioning material by means of a transfer film of pressure sensitive adhesive.

10. A shoe insert according to Claim 1 in which the surface receiving the fastening material includes an index scale extending from the toe end to the heel end for positioning the cushion pad elements.

11. A shoe insert according to Claim 1 in which the cushion pad elements comprising a layer of cushioning material are attached to a sheet of fastening material.

12. A shoe insert according to claim 11 in which a plurality of pads are provided and at least one element contains a pad having a thicker cushioning layer than other pads.

13. A shoe insert according to Claim 11 in which the edges of the cushioning material are shaped.

14. A shoe insert according to Claim 11 in which the cushioning pad includes a cavity to relieve pressure on sensitive areas of the foot.

15. A shoe insert according to Claim 14 in which the edges of the cavity are shaped.

16. A shoe insert according to Claim 11 in which the cushioning layer is inclined so as to be thicker from one side to the other whereby pronation of the foot of the user is adjusted.

17. A shoe insert according to Claim 11 in which both surfaces of at least one of the cushioning pads contain fastening material.

18. A shoe insert according to Claim 11 in which both surfaces of the shoe insert contains loop fabric.

19. A shoe insert according to Claim 1 containing at least one shoe insert and a plurality of pads of different thicknesses and shapes enclosed in a package.
